(19) Europäisches Patentamt
European Patent Office
Office européen des brevets

(11) Veröffentlichungsnummer : **0 014 177**
**B1**

(12) # EUROPÄISCHE PATENTSCHRIFT

(45) Veröffentlichungstag der Patentschrift : **07.04.82**

(51) Int. Cl.³ : **C 07 C143/40**, C 07 C139/00, D 06 L 3/12

(21) Anmeldenummer : **80810011.9**

(22) Anmeldetag : **11.01.80**

(54) **Sulfogruppenhaltiges Bis-styryl-benzol, dessen Herstellung und Verwendung zum optischen Aufhellen organischer Fasermaterialien.**

(30) Priorität : **17.01.79 CH 439/79**

(43) Veröffentlichungstag der Anmeldung : **06.08.80 (Patentblatt 80/16)**

(45) Bekanntmachung des Hinweises auf die Patenterteilung : **07.04.82 Patentblatt 82/14**

(84) Benannte Vertragsstaaten : **CH DE FR GB IT**

(56) Entgegenhaltungen :
**DE - A - 1 923 267**
**DE - A - 2 525 679**
**FR - A - 2 139 387**
**FR - A - 2 168 210**

(73) Patentinhaber : **CIBA-GEIGY AG**
**Patentabteilung Postfach**
**CH-4002 Basel (CH)**

(72) Erfinder : **Weber, Kurt, Dr.**
**Rennweg 98**
**CH-4052 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 014 177 B1

## Sulfogruppenhaltiges Bis-styryl-benzol, dessen Herstellung und Verwendung zum optischen Aufhellen organischer Fasermaterialien

Die vorliegende Erfindung betrifft ein neues sulfogruppenhaltiges Bis-styrylbenzol.

Es sind bereits verschiedene sulfogruppenhaltige Bis-styrylbenzole bekannt, so zum Beispiel aus der US-PS 3,904,678.

Es wurde nun überraschenderweise gefunden, dass ein sulfogruppenhaltiges Bis-styrylbenzol besonders gute Aufhelleffekte ergibt.

Die vorliegende Erfindung betrifft demnach das sulfogruppenhaltige Bis-styrylbenzol der Formel

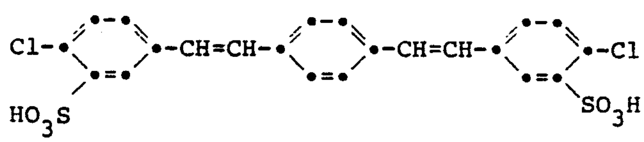

(I)

sowie dessen Salze.

Unter Salze sind Erdalkalimetallsalze, zum Beispiel Calcium-, Barium- oder Magnesiumsalze sowie insbesondere Alkalimetallsalze, z.B. Natrium- oder Kaliumsalze aber auch Ammonium- oder Aminsalze bevorzugt.

Die neue Verbindung wird vorteilhaft hergestellt indem man ein Mol der Verbindung der Formel

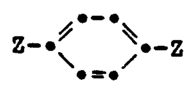

(II)

worin Z die Gruppierung

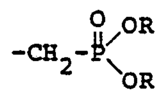

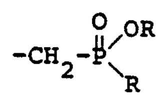

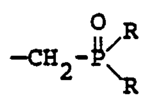

oder

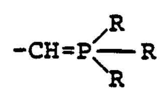

worin R einen unsubstituierten oder substituierten Alkylrest, einen Arylrest, einen Cycloalkylrest oder einen Aralkylrest darstellt, mit 2 Mol der Verbindung der Formel

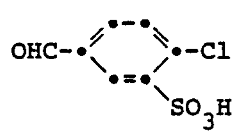

(III)

oder eines ihrer Alkalimetall-, Ammonium- oder Aminsalze umsetzt und gewünschtenfalls die erhaltene Verbindung in an sich bekannter Weise in die freie Säure oder deren Alkalimetall-, Erdalkalimetall-, Ammonium- und Aminsalze überführt.

Die vorstehend definierten neuen Verbindungen zeigen in gelöstem oder feinverteiltem Zustande eine mehr oder weniger ausgeprägte Fluoreszenz. Sie können zum optischen Aufhellen der verschiedensten synthetischen, halbsynthetischen oder natürlichen organischen Materialien oder Substanzen, welche solche organische Materialien enthalten, verwendet werden.

Hierfür seien beispielsweise, ohne dass durch die nachfolgende Uebersicht irgendeine Beschränkung hierauf ausgedrückt werden soll, die folgenden Gruppen von organischen Materialien, soweit eine optische Aufhellung derselben in Betracht kommt, genannt :

I. Synthetische organische hochmolekulare Materialien :

a) Polymerisationsprodukte auf Basis mindestens eine polymerisierbare Kohlenstoff-Kohlenstoff-Doppelbindung enthaltender organischer Verbindungen, d.h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von α,β-ungesättigten Carbonsäuren oder Derivaten solcher Carbonsäuren, insbesondere von Acrylverbindungen (wie z.B. Acrylestern, Acrylsäure, Acrylnitril, Acrylamiden und deren Derivaten oder deren Methacryl-Analoga), von Olefin-Kohlenwasserstoffen (wie z.B. Aethylen, Propylen, Styrole oder Diene, ferner sogenannte ABS-Polymerisate), Polymerisate auf Basis von Vinyl-und Vinyliden-Verbindungen (wie z.B. Vinylchlorid, Vinylalkohol, Vinylidenchlorid),

b) Polymerisationsprodukte, die durch Ringöffnung erhältlich sind, z.B. Polyamide vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Aethylenglykolterephthalsäure-Polyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide (z.B. Hexamethylendiaminadipat), Maleinatharze, Melaminharze, deren Vorkondensate und Analoga, Polycarbonate, Silikone,

d) Polyadditionsprodukte wie Polyurethane (vernetzt und unvernetzt), Epoxidharze,

II. Halbsynthetische organische Materialien, z.B. Cellulose-ester verschiedener Veresterungsgrade (sogenanntes 2 1/2-Acetat, Triacetate) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen wie Baumwolle, Wolle, Leinen, Seide, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d.h. beispielsweise als vorwiegend dreidimensional ausgedehnte Körper wie Platten, Profile, Spritzgussformlinge, verschiedenartige Werkstücke, Schnitzel, Granulate oder Schaumstoffe, ferner als vorwiegend zweidimensional ausgebildete Körper wie Filme, Folien, Lacke, Ueberzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z.B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäss anzuwendenden Verbindungen kommt u.a. Bedeutung für die Behandlung von textilen organischen Materialien, insbesondere textilen Geweben, zu. Sofern Fasern, welche als Stapelfasern oder Endlosfäden, in Form von Strangen, Geweben, Gewirken, Vliesen, beflockten Substraten oder Verbundstoffen vorliegen können, erfindungsgemäss optisch aufzuhellen sind, geschieht dies mit Vorteil in wässrigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier- Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140 °C, beispielsweise bei Siedetemperatur des Bades oder in deren Nähe (etwa 90 °C), durchgeführt. Für die erfindungsgemässe Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäss vorliegender Erfindung können ferner den Materialien vor oder während deren Verformung zugesetzt bzw. einverleibt werden.

Sofern die Formgebung voll- oder halbsynthetischer organischer Materialien durch Spinnverfahren bzw. über Spinnmassen erfolgt, können die optischen Aufheller nach folgenden Verfahren appliziert werden :

— Zugabe zu den Ausgangssubstanzen (z.B. Monomeren) oder Zwischenprodukten (z.B. Vorkondensaten, Praepolymeren), d.h. vor oder während der Polymerisation, Polykondensation oder Polyaddition,

— Aufpudern auf Polymerisatschnitzel oder Granulate für Spinnmassen,

— Badfärbung von Polymerisatschnitzeln oder Granulaten für Spinnmassen,

— Dosierte Zugabe zu Spinnschmelzen oder Spinnlösungen,

— Applikation auf Spinnkabel vor dem Verstrecken.

Die neuen optischen Aufhellmittel gemäss vorliegender Erfindung können beispielsweise auch in folgenden Anwendungsformen eingesetzt werden :

a) Mischungen mit Farbstoffen (Nuancierung) oder Pigmenten (Farb- oder insbesondere z.B. Weisspigmenten) oder als Zusatz zu Färbebädern, Druck-, Aetz- oder Reservepasten. Ferner auch zur Nachbehandlung von Färbungen, Drucken oder Aetzdrucken,

b) in Mischungen mit sogenannten « Carriern », Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),

c) in Mischungen mit Vernetzern, Appreturmitteln (z.B. Stärke oder synthetischen Appreturen) sowie in Kombination mit den verschiedensten Textilveredlungsverfahren, insbesondere Kunstharzausrüstungen (z.B. Knitterfest-Ausrüstungen wie « wash-and-wear », « permanent-press », « no-iron »), ferner Flammfest-, Weichgriff-, Schmutzablöse (« anti-soiling »)- oder Antistatisch-Ausrüstungen oder antimikrobiellen Ausrüstungen,

d) Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Vliese, Papier, Leder,

e) als Zusätze zu sogenannten « master batches »,

f) als Zusätze zu den verschiedensten industriellen Produkten, um dieselben marktfähiger zu machen (z.B. Aspektverbesserung von Seifen, Waschmitteln, Pigmenten),

g) in Kombination mit anderen, optisch aufhellend wirkenden Substanzen,

h) in Spinnbadpräparationen, d.h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,

i) als Szintillatoren, für verschiedene Zwecke photographischer Art, wie z.B. für elektrophotographische Reproduktion oder Supersensibilisierung,

j) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische (z.B. Hitze) oder eine kombinierte chemisch/thermische Behandlung darstellen. So verfährt man beispielsweise bei der optischen Aufhellung einer Reihe von Fasersubstraten, mit den erfindungsgemässen Aufhellern zweckmässig in der Weise, dass man diese Fasern mit den wässrigen Lösungen (gegebenenfalls auch Dispersionen) der Aufhellmittel bei Temperaturen unter 75 °C, z.B. bei Raumtemperatur, imprägniert und einer trockenen Wärmebehandlung bei Temperaturen über 100 °C unterwirft, wobei es sich im allgemeinen empfiehlt, das Fasermaterial vorher noch bei mässig erhöhter Temperatur, z.B. bei mindestens 60 °C bis etwa 130 °C zu trocknen. Die Wärmebehandlung in trockenem Zustande erfolgt dann vorteilhaft bei Temperaturen zwischen 120 und 225 °C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einen einzigen Arbeitsgang zusammengelegt werden.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,000 1 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,000 5 und 0,5 Gewichtsprozent von Interesse.

Aus verschiedenen Gründen ist es oft zweckmässig, die Aufheller nicht als solche, d.h. rein einzusetzen, sondern vermischt mit den verschiedensten Hilfs- und Coupiermitteln, wie z.B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumtripolyphosphaten oder Alkalimetallsilicaten.

Die neuen optischen Aufhellmittel eignen sich auch besonders als Zusätze für Waschbäder oder zu Gewerbe- und Haushaltwaschmitteln, wobei sie in verschiedener Weise zugesetzt werden können. Zu Waschbädern werden sie zweckmässig in Form ihrer Lösungen in Wasser oder organischen Lösungsmitteln oder auch in feiner Verteilung als wässrige Dispersionen zugegeben. Zu Haushalt- oder gewerblichen Waschmitteln werden sie vorteilhaft in irgendeiner Phase des Herstellungsprozesses der Waschmittel, z.B. der sogenannten « slurry » vor dem Zerstäuben dem Waschpulver oder bei der Vorbereitung flüssiger Waschmittelkombinationen, zugesetzt. Die Zugabe kann sowohl in Form einer Lösung oder Dispersion in Wasser oder anderen Lösungsmitteln als auch ohne Hilfsmittel als trockenes Aufhellerpulver erfolgen. Man kann die Aufhellmittel beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen wie beispielsweise Seife in Form von Schnitzeln und Pulver, Synthetika, lösliche Salze von Sulfonsäurehalbestern höherer Fettalkohole, höher und/oder mehrfach alkylsubstituierten Arylsulfonsäuren, Sulfocarbonsäureester mittlerer bis höherer Alkohole, Fettsäureacylaminoalkyl- oder -aminoarylglycerinsulfonate, Phosphorsäureester von Fettalkoholen usw. in Frage. Als Aufbaustoffe, sogenannte « Builders », kommen z.B. Alkalipoly- und -polymetaphosphate, Alkalipyrophosphate, Alkalisalze der Carboxymethylcellulose und andere « Soilredepositionsinhibitoren », ferner Alkalisilikate, Alkalicarbonate, Alkaliborate, Alkaliperborate, Nitrilotriessigsäure, Aethylendiaminotetraessigsäure, Schaumstabilisatoren wie Alkanolamide höherer Fettsäuren, in Betracht. Ferner können in den Waschmitteln beispielsweise enthalten sein : antistatische Mittel, rückfettende Hautschutzmittel wie Lanolin, Enzyme, Antimikrobika, Parfüme und Farbstoffe.

Die neuen optischen Aufheller haben den besonderen Vorteil, dass sie auch bei Gegenwart von Aktivchlorspendern, wie z.B. Hypochlorit, wirksam sind und ohne wesentliche Einbusse der Effekte in Waschbädern mit nichtionogenen Waschmitteln, z.B. Alkylphenolpolyglykoläthern, verwendet werden können.

Die erfindungsgemässen Verbindungen werden in Mengen von 0,005 bis 1 % oder mehr, bezogen auf das Gewicht des flüssigen oder pulverförmigen, fertigen Waschmittels, zugesetzt. Waschflotten, die die angegebenen Mengen der beanspruchten Aufheller enthalten, verleihen beim Waschen von Textilien aus Cellulosefasern, Polyamidfasern, hochveredelten Cellulosefasern, Polyesterfasern, Wolle etc. diesen einen brillanten Aspekt am Tageslicht.

Die Waschbehandlung wird beispielsweise wie folgt durchgeführt :

Die angegebenen Textilien werden während 1 bis 30 Minuten bei 20 bis 100 °C in einem Waschbad behandelt, das 1 bis 10 g/kg eines aufgebauten, zusammengesetzten Waschmittels und 0,05 bis 1 %, bezogen auf das Waschmittelgewicht, der beanspruchten Aufhellmittel enthält. Das Flottenverhältnis kann 1 : 3 bis 1 : 50 betragen. Nach dem Waschen wird wie üblich gespült und getrocknet. Das Waschbad kann als Bleichzusatz 0,2 g/l Aktivchlor (z.B. als Hypochlorit) oder 0,1 bis 2 g/l Natriumperborat enthalten.

In den Beispielen sind Prozente immer Gewichtsprozente.

## Beispiel 1

18,9 g p-Xylylen-diphosphonsäure-tetraäthylester und 25,2 g 4-Chlorbenzaldehyd-3-sulfonsäure, Natriumsalz (Gehalt : 96 %) werden in 200 ml Dimethylsulfoxid bei 40 °C unter Verdrängung der Luft durch einen Stickstoffstrom, gelöst. Innert 20 Minuten werden dann 7,2 g Natriummethylat (Gehalt : 97,3 %) bei 40 bis 45 °C eingetragen und noch 4 Stunden lang bei 40 bis 45 °C nachgerührt. Dann werden unter Kühlung 100 ml entsalztes Wasser zugegeben. Bei 15 °C wird das auskristallisierte Produkt abgenutscht, mit 100 ml entsalztem Wasser gewaschen und zweimal aus 2 000 ml bzw. 1 500 ml Wasser umkristallisiert. Nach Trocknung unter Vakuum bei 85 °C erhält man 15,3 g der Verbindung der Formel

(100) 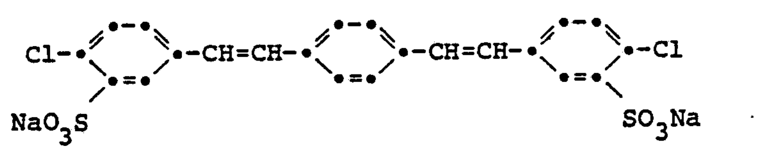

als blassgelbes Pulver. UV-Sepktrum : λ max : 363 nm gemessen in Dimethylformamid/Wasser 1 : 1.

Anstelle von p-Xylylen-diphosphonsäure-tetraäthylester kann auch die äquivalente Menge p-Xylylendiphosphonsäure-tetramethylester verwendet werden. Ebenso kann anstelle von Dimethylsulfoxid, Dimethylformamid und anstelle von festem Natriummethylat eine ca. 30 %ige methanolische Lösung von Natriummethylat verwendet werden. Schliesslich kommen als alkalische Kondensationsmittel auch Natrium- oder Kaliumhydroxidpulver in Frage.

Durch Lösen der Verbindung der Formel (1) in Wasser und Zugabe von Barium- bzw. Calciumchlorid erhält man das entsprechende Barium- bzw. Calciumsalz.

Lässt man die wässrige Lösung der Verbindung der Formel (1) durch eine Säule, welche einen stark sauren Ionenaustauscher enthält, fliessen, erhält man, nach dem Eindampfen der nun sauren Lösung zur Trockne, die Verbindung der Formel (1) in Form ihrer freien Sulfonsäure. Durch Neutralisation der wässrigen Lösung der freien Sulfonsäure mit äquimolaren Mengen derentsprechenden Base können die in der folgenden Tabelle aufgeführten Verbindungen erhalten werden.

(101) 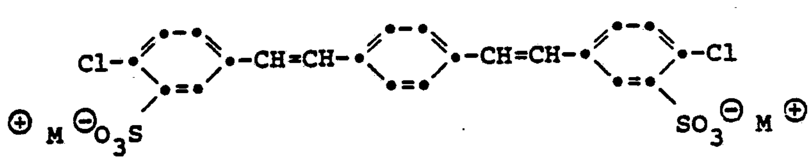

Tabelle I

| Verbindung Nr. | $M^{\oplus}$ |
|---|---|
| 102 | K |
| 103 | $NH_4$ |
| 104 | $HN(CH_2CH_2OH)_3$ |
| 105 | 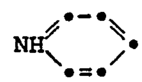 |
| 106 | $H_2N(CH_2CH_2OH)_2$ |
| 107 | $H_3NCH_2CH_2OH$ |
| 108 | $H_2N(CH_3)_2$ |
| 109 | $H_3N-CH_3$ |

Beispiel 2

Ein Polyamid 6.6-Webtrikot wird auf einem Färbeapparat bei einem Flottenverhältnis von 1 : 30 mit einem wässrigen Bad folgender Zusammensetzung behandelt : 0,5 % der Verbindung der Formel (100) bezogen auf das Warengewicht und 3 g pro Liter Natriumdithionit ($Na_2S_2O_4 \cdot 2H_2O$) stabilisiert mit Natriumpyrophosphat ($Na_2P_2O_7$) und 1 ml pro Liter Essigsäure 80 %.

Die Applikation erfolgt gemäss folgendem Temperatur-programm :

40 °C bis 97 °C innerhalb 30 Minuten
97 °C während 30 Minuten und
97 °C bis 40 °C innerhalb 15 Minuten.

Zur Nachbehandlung wird das Textilgut während 30 Sekunden in fliessendem, deionisiertem Wasser gespült. Das so behandelte Polyamid 6.6-Webtrikot weist nach dem Trocknen einen hohen Aufhelleffekt auf.

Beispiel 3

Man foulardiert bei Raumtemperatur ein Polyamid 6-Webtrikot (nicht fixiert) mit folgender wässeriger Flotte :

4 g pro Liter der Verbindung der Formel (100)
16 g pro Liter Harnstoff
15 g pro Liter Polyoxyäthylen mit einem Mol Gewicht von 600
5 ml pro Liter Essigsäure, 80 %.

Der Abquetscheffekt beträgt 100 %. Anschliessend wird 30 Sekunden bei 190 °C thermofixiert. Das so behandelte Trikot weist einen hohen Aufhelleffekt auf.

Beispiel 4

Ein Baumwollgewebe wird auf einem Färbeapparat bei einem Flottenverhältnis von 1 : 30 mit einem wässerigen Bad folgender Zusammensetzung behandelt :

0,2 % der Verbindung der Formel (100)
5 g pro Liter Glaubersalz krist. (Zugabe nach 15 Minuten).

Die Applikation erfolgt gemäss folgendem Temperatur-programm :

20 °C bis 50 °C innerhalb 15 Minuten
50 °C während 15 Minuten.

Zur Nachbehandlung wird das Textilgut während 30 Sekunden in fliessendem, deionisiertem Wasser gespült.

Das so behandelte Baumwollgewebe weist nach dem Trocknen einen hohen Aufhelleffekt auf.

Beispiel 5

Man foulardiert bei Raumtemperatur ein Baumwollgewebe mit einer wässrigen Flotte, die pro Liter 2 g der Verbindung der Formel (100) enthält. Der Abquetscheffekt beträgt 75 %. Anschliessend wird 60 Sekunden bei 130 °C getrocknet. Das so behandelte Baumwollgewebe weist einen hohen Aufhelleffekt auf.

Beispiel 6

Baumwoll-Textilmaterial wird im Flottenverhältnis 1 : 20 während 15 Minuten in einer 60 °C warmen Flotte gewaschen, die pro Liter 4 g eines Waschpulvers folgender Zusammensetzung enthält :

0,8 % der Verbindung der Formel (100)
15,7 % Alkylarylsulfonat
3,7 % Fettalkoholsulfonat
2,7 % Kokossäuremonoäthanolamid
39,0 % Na-Tripolyphosphat
4,0 % Na-Silikat
2,0 % Magnesiumsilikat
1,0 % Carboxymethylcellulose
0,5 % Aethylendiamintetraessigsäure-Natriumsalz

Rest Natriumsulfat und Wasser.

Nach dem Spülen und Trocknen weist das Baumwoll-Textilmaterial einen schönen Aufhelleffekt auf.

**Ansprüche**

1. Das Bis-styryl-benzol der Formel

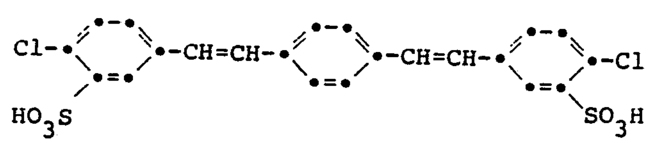

und dessen Alkalimetall-, Erdalkalimetall-, Ammonium- und Aminsalze.

2. Das Bis-styryl-benzol gemäss Anspruch 1 der Formel

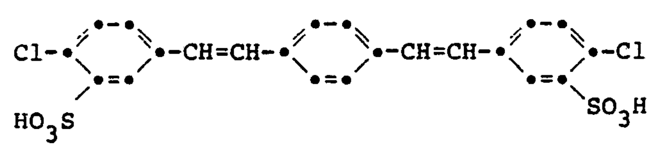

und dessen Natrium- und Kaliumsalze.

3. Das Bis-styryl-benzol gemäss Anspruch 2 der Formel

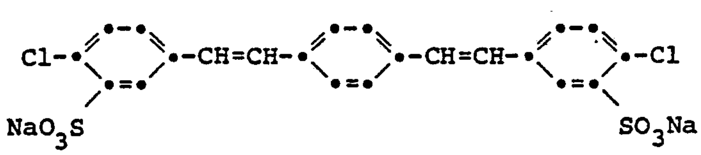

4. Verfahren zur Herstellung des Bis-styryl-benzols der formel

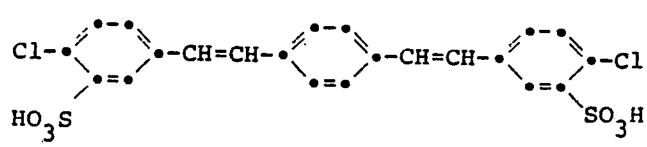 (I)

und dessen Alkalimetall-, Erdalkalimetall-, Ammonium- und Aminsalze, dadurch gekennzeichnet, dass man 1 Mol der Verbindung der Formel

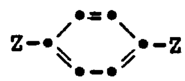

(II)

worin Z die Gruppierung der Formel

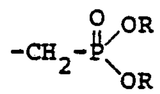

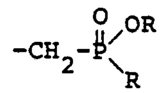

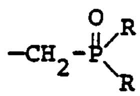

oder

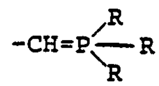

worin R einen unsubstituierten oder substituierten Alkylrest, einen Arylrest, einen Cycloalkylrest oder einen Aralkylrest bedeutet, mit 2 Mol der Verbindung der Formel

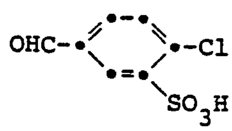

(III)

oder eines ihrer Alkalimetall-, Ammonium- oder Aminsalze umsetzt und gewünschtenfalls die erhaltene Verbindung in an sich bekannter Weise in die freie Säure oder deren Alkalimetall-, Erdalkalimetall-, Ammonium- und Aminsalze überführt.

5. Verwendung des im Anspruch 1 definierten Bis-styrylbenzols zum optischen Aufhellen von organischen Fasermaterialien.

6. Verwendung gemäss Anspruch 5 zum optischen Aufhellen von Cellulose und Polyamiden als organischen Fasermaterialien.

**Claims**

1. The bis-styrylbenzene of the formula

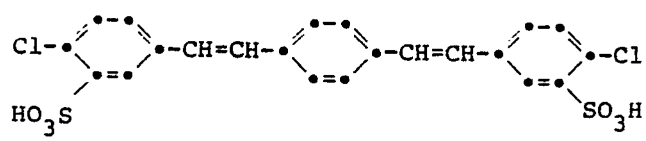

and the alkali metal, alkaline earth metal, ammonium and amine salts thereof.

2. The bis-styrylbenzene according to claim 1 of the formula

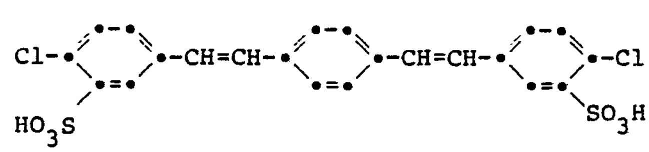

and the sodium and potassium salts thereof.

3. The bis-styrylbenzene according to claim 2 of the formula

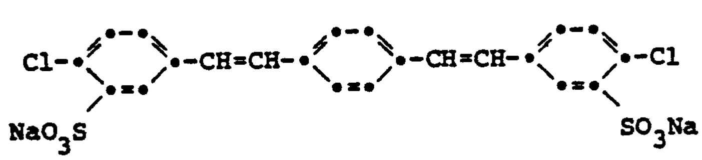

4. A process for the production of the bis-styrylbenzene of the formula

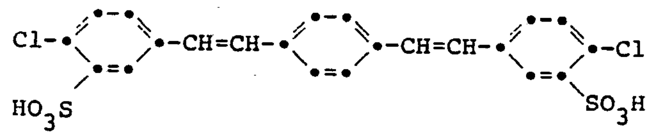

(I)

and the alkali metal, alkaline earth metal, ammonium and amine salts thereof, which process comprises reacting 1 mole of the compound of the formula

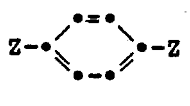

(II)

wherein Z is the grouping of the formula

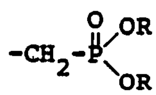

,

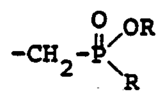

,

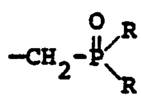

or

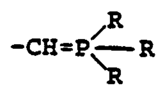

wherein R is an unsubstituted or substituted alkyl radical, an aryl radical, a cycloalkyl radical or an aralkyl radical, with 2 moles of the compound of the formula

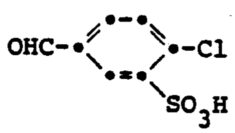

(III)

or of an alkali metal, ammonium or amine salt thereof, and, if desired, converting the resultant compound, in a manner known per se, into the free acid or the alkali metal, alkaline earth metal, ammonium and amine salts thereof.

5. Use of the bis-styrylbenzene defined in claim 1 for whitening organic fibre materials.

6. Use according to claim 5, wherein the organic fibre materials to be whitened are cellulose and polyamide.

**Revendications**

1. Bis-styryl-benzène de formule

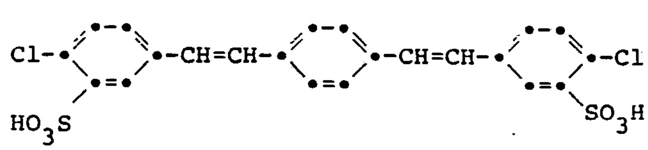

et ses sels de métaux alcalins, de métaux alcalinoterreux, d'ammonium et d'amines.

2. Bis-styryl-benzène selon la revendication 1, de formule

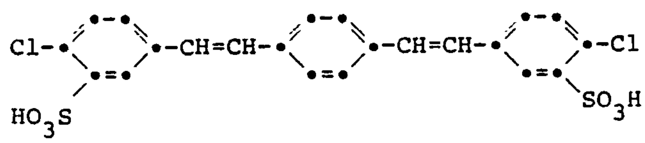

et ses sels de sodium et de potassium.

3. Bis-styryl-benzène selon la revendication 2, de formule

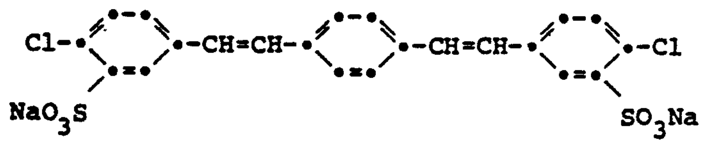

4. Procédé pour la préparation du bis-styryl-benzène de formule

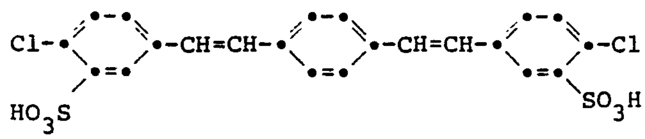 (I)

et ses sels de métaux alcalins, de métaux alcalinoterreux, d'ammonium et d'amines, caractérisé par le fait que l'on fait réagir 1 mole du composé de formule

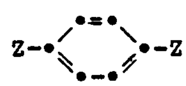 (II)

où Z représente le groupement de formule

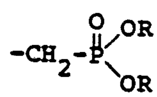 , 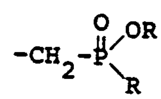 ,

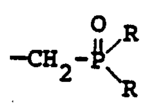 ou 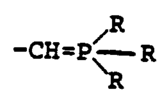

où R représente un reste alcoyle non substitué ou substitué, un reste aryle, un reste cycloalcoyle ou un reste aralcoyle, avec 2 moles du composé de formule

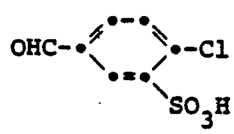 (III)

ou un de ses sels de métaux alcalins, d'ammonium ou d'amine, et, si on le désire, on transforme le composé obtenu, de manière connue en soi, en l'acide libre ou en ses sels de métaux alcalins, de métaux alcalinoterreux, d'ammonium ou d'amine.

5. Utilisation du bis-styryl-benzène défini dans la revendication 1 pour l'azurage optique des matières fibreuses organiques.

6. Utilisation selon la revendication 5 pour l'azurage optique de la cellulose et des polyamides comme matières fibreuses organiques.